# FASCICULE DE BREVET EUROPEEN

(11) **EP 0 747 337 B1**
(45) Date de publication et mention de la délivrance du brevet: **27.12.2000**
(21) Numéro de dépôt: 96201527.7
(22) Date de dépôt: 31.05.1996
(51) Int. Cl.: C07C 17/23, C07C 21/18, B01J 23/89

(54) **Procédé pour la préparation de chlorotrifluoroéthylène et de trifluoroéthylène au départ de 1,1,2-trichloro-1,2,2-trifluoroéthane et composition catalytique utilisée dans ce procédé**
Verfahren zur Herstellung von Chlortrifluorethylen und von Trifluorethylen aus 1,1,2-Trichlor-1,2,2-trifluorethan sowie katalytische Zusammensetzung zur Verwendung in diesem Verfahren
Process for the preparation of chlorotrifluoroethylene and of trifluoroethylene from 1,1,2-trichloro-1,2,2-trifluoroethane and catalytic composition used in this process

(30) Priorité: 06.06.1995 US 470993
(43) Date de publication de la demande: 11.12.1996
(73) Titulaire: SOLVAY (Société Anonyme), 1050 Bruxelles (BE)
(72) Inventeur: Lerot, Luc, 1200 Bruxelles (BE); Wilmet, Vincent, 1301 Wavre (BE); Pirotton, Joseph, 1030 Bruxelles (BE)
(74) Mandataire: Jacques, Philippe

(56) Documents cités:
- EP-A- 0 496 446
- EP-A- 0 556 893
- WO-A-89/00452

## Description

La présente invention concerne un procédé pour la préparation de chlorotrifluoroéthylène et de trifluoroéthylène au départ de 1,1,2-trichloro-1,2,2-trifluoroéthane, ainsi qu'une composition catalytique utilisée dans ce procédé.

Le chlorotrifluoroéthylène et le trifluoroéthylène sont des monomères intéressants pour la fabrication de nombreux polymères, homo- et copolymères, fluorés et chlorofluorés.

Dans les demandes de brevet européen EP-A-0 459 463, EP-A- 0 485 246 et EP-A- 0 496 446, on décrit la préparation de fluoroéthylènes et de chlorofluoroéthylènes par réaction en phase gazeuse de 1,1,2-trichloro-1,2,2-trifluoroéthane en présence d'hydrogène et de catalyseurs supportés sur carbone constitués de mélanges de cuivre et d'un métal du groupe VIII. Toutefois, toutes les compositions catalytiques décrites contiennent moins de 10 % de cuivre, par rapport au poids total de la composition catalytique.

La présente invention a pour but de procurer un procédé pour la préparation de chlorotrifluoroéthylène et de trifluoroéthylène au départ de 1,1,2-trichloro-1,2,2-trifluoroéthane avec des compositions catalytiques de stabilité notablement améliorée.

A cet effet, l'invention concerne un procédé pour la préparation de chlorotrifluoroéthylène et de trifluoroéthylène par réaction en phase gazeuse de 1,1,2-trichloro-1,2,2-trifluoroéthane en présence d'hydrogène et d'une composition catalytique comprenant un support à base de carbone sur lequel sont déposés d'environ 12 à environ 22 % en poids de cuivre par rapport au poids total de la composition catalytique et au moins un métal du groupe VIII du tableau périodique des éléments.

On travaille avantageusement avec d'environ 13,5 à environ 20 % en poids de cuivre. De manière particulièrement préférée, on travaille avec d'environ 16 à environ 19 % en poids de cuivre, par rapport au poids total de la composition catalytique.

Le procédé selon l'invention offre d'excellents résultats se traduisant notamment par un taux de transformation de 1,1,2-trichloro-1,2,2-trifluoroéthane généralement supérieur à 75 % en poids et par une grande stabilité des compositions catalytiques. Il offre également une excellente sélectivité en chlorotrifluoroéthylène et en trifluoroéthylène, généralement supérieure à 90 % en poids. Il permet enfin de moduler, en fonction des conditions de réaction, les proportions respectives de chlorotrifluoroéthylène et de trifluoroéthylène dans le mélange produit.

Un effet surprenant de la présente invention réside dans le fait que la mise en oeuvre d'une composition catalytique comprenant de faibles quantités de cuivre présente une stabilité catalytique insuffisante, alors que le procédé selon l'invention procure une stabilité remarquable de la composition catalytique, permettant de maintenir pendant un temps très long à la fois un taux de transformation élevé du 1,1,2-trichloro-1,2,2-trifluoroéthane (c'est-à-dire une activité catalytique élevée) et une sélectivité élevée en chlorotrifluoroéthylène et en trifluoroéthylène (c'est-à-dire une production négligeable de sous-produits hydrogénés).

Parmi les métaux du groupe VIII du tableau périodique des éléments, utilisables seuls ou en mélange, on donne la préférence au ruthénium, au rhodium, à l'iridium, au platine et au palladium et plus particulièrement encore à l'iridium, au platine et au palladium. Les métaux du groupe VIII du tableau périodique des éléments tout particulièrement préférés sont le platine et le palladium.

Le procédé selon l'invention met en oeuvre une composition catalytique qui comprend au moins 0,05 % et, par ailleurs, pas plus de 10 % en poids de métal du groupe VIII du tableau périodique des éléments, par rapport au poids total de la composition catalytique. De préférence, elle comprend de 1 % à 5 % en poids de métal du groupe VIII du tableau périodique des éléments, par rapport au poids total de la composition catalytique.

Le rapport en poids entre le cuivre et le métal du groupe VIII du tableau périodique des éléments dans la composition catalytique peut varier dans de larges limites. Le plus souvent, il est d'au moins 0,1 sans excéder 20. On travaille avantageusement avec un rapport d'au moins 0,4. De préférence, on ne dépasse pas un rapport de 10.

Le cuivre et le métal (ou les métaux) du groupe VIII du tableau périodique des éléments sont généralement mis en oeuvre dans le procédé selon l'invention sous forme de composés organiques ou inorganiques. Comme composés organiques utilisables, on peut mentionner les carboxylates, les alcoolates et les acétylacétonates, ayant une chaîne alkyle qui contient de 1 à 10 atomes de carbone. Comme composés inorganiques utilisables, on peut mentionner les halogénures, les hydroxydes et les nitrates et plus particulièrement, les halogénures et les hydroxydes. De manière avantageuse, on choisit les chlorures, fluorures et hydroxydes. On obtient d'excellents résultats avec les chlorures.

Le support à base de carbone est généralement constitué de charbon actif ayant un important volume poreux. Le plus souvent, ce volume poreux est compris entre 0,1 et 2 cm³/g. Il est de préférence compris entre 0,2 et 1 cm³/g.

La surface spécifique du support à base de carbone est généralement comprise entre 10 et 1.500 m²/g.

Le procédé selon l'invention met en oeuvre une composition catalytique qui peut être obtenue par imprégnation du support avec les solutions de composés métalliques. L'imprégnation du support peut être réalisée par n'importe quelle méthode. En pratique, elle peut être obtenue par la technique dite du "volume poreux" (imprégnation dite "sèche") ou par la technique du "volume excédentaire" (imprégnation dite "humide"). De telles méthodes sont décrites dans la littérature, en particulier par Charles N. Satterfield "Heterogeneous catalysis in practice", 1980, Mc Graw-Hill, New-York, spécialement p. 82 et 83, qui est incorporé à la présente demande par référence.

Les solutions d'imprégnation peuvent être aqueuses ou organiques. De préférence, on met en oeuvre une solution aqueuse ou alcoolique.

L'ordre d'imprégnation du support n'est pas critique. L'imprégnation peut donc être réalisée d'abord avec une solution comprenant le cuivre ou encore d'abord avec une solution comprenant au moins un métal du groupe VIII du tableau périodique des éléments, ou simultanément avec ces deux solutions. De préférence, on imprègne d'abord avec une solution comprenant le métal présent en quantité prépondérante.

Après imprégnation, le support peut être séché avant d'être introduit dans le réacteur proprement dit.

La composition catalytique ainsi obtenue peut être mise en oeuvre dans le procédé selon l'invention telle quelle ou peut être préalablement réduite soit par de l'hydrogène, soit par un mélange d'hydrogène avec un gaz inerte tel que l'hélium.

La température à laquelle s'effectue la réaction selon le procédé de l'invention se situe habituellement entre 80 et 600°C. De préférence, cette température se si tue entre 120 et 400°C et plus particulièrement encore entre 200 et 300°C.

La pression sous laquelle est effectuée la réaction n'est pas critique en elle-même. Habituellement, on opère sous une pression comprise entre 1x10⁵ et 10x10⁵ pascals. De préférence, on retient une pression comprise entre 2x10⁵ et 5x10⁵ pascals.

Le rapport volumique entre l'hydrogène et le 1,1,2-trichloro-1,2,2-trifluoroéthane mis en oeuvre est généralement compris entre 0,25 et 20. De préférence, il est d'au moins 0,4 et ne dépasse pas 10. Suivant un mode de réalisation particulièrement préféré, ce rapport est compris entre 1 et 7. C'est ainsi qu'avec un rapport situé aux environs de 4, d'excellents résultats ont été obtenus qui se traduisent en particulier par une très grande stabilité de la composition catalytique.

Le temps de contact moyen entre la composition catalytique et les produits en réaction est généralement d'au moins 0,5 secondes et ne dépasse pas 10 secondes. De préférence, il est d'au moins 3 secondes et ne dépasse pas 7 secondes.

Le procédé selon l'invention peut être réalisé en présence d'un gaz inerte tel que par exemple l'hélium.

Le procédé selon l'invention peut être mis en oeuvre dans tous types de réacteurs. En particulier, il peut aussi bien être mis en oeuvre dans un réacteur à lit fixe que dans un réacteur à lit fluidisé.

L'invention concerne également une composition catalytique pour la préparation de chlorotrifluoroéthylène et de trifluoroéthylène par réaction en phase gazeuse de 1,1,2-trichloro-1,2,2-trifluoroéthane en présence d'hydrogène, laquelle comprend un support à base de carbone sur lequel sont déposés du cuivre et au moins un métal du groupe VIII du tableau périodique des éléments.

Les exemples non limitatifs qui suivent sont donnés dans le but d'illustrer l'invention. L'exemple 1 est donné à titre de référence. Les exemples 2 à 5 illustrent la présente invention.

### Exemples 1-5

Différentes compositions catalytiques comprenant du cuivre et du palladium déposés sur un support à base de carbone ont été préparées suivant le protocole décrit à l'exemple 1 de la demande de brevet EP-A- 0 496 446. La composition pondérale des différentes compositions catalytiques testées est donnée dans le tableau I.

L'hydrogénation du 1,1,2-trichloro-1,2,2-trifluoroéthane (CFC-113) a été réalisée dans le réacteur décrit à l'exemple 1 de la demande de brevet EP-A- 0 496 446, dans les conditions opératoires suivantes :
- température : 300 °C;
- pression : 3 x 10⁵ Pa;
- débit de CFC-113 : 8 cm³/min;
- débit d'hydrogène : 12 cm³/min;
- temps de séjour moyen : 4,3 s.

Dans ces conditions opératoires, le taux de conversion initial du CFC-113 est quasi de 100 %. Le taux de conversion du CFC-113 décroit cependant au cours du temps, reflétant une baisse d'activité du catalyseur.

La durée de réaction avant d'atteindre des taux de conversion du CFC-113 donnés et la quantité globale de CFC-113 transformé avant que le taux de conversion n'atteigne 80 % sont rassemblées au tableau I.

Ces exemples démontrent la stabilité accrue des compositions catalytiques selon l'invention dans le procédé de fabrication de chlorotrifluoroéthylène et de trifluoroéthylène au départ de CFC-113 et d'hydrogène.

**Tableau I**

| | 1 | 2 | 3 | 4 | 5 |
|---|---|---|---|---|---|
| Teneur en métal dans la composition catalytique | | | | | |
| Cu (% poids) | 8,1 | 13,0 | 17,5 | 18,4 | 20,7 |
| Pd (% poids) | 2,1 | 2,1 | 1,5 | 1,6 | 2,8 |
| durée de réaction (h) avant d'atteindre un taux de conversion du CFC-113 de | | | | | |
| 95 % | 7 | 15 | n.m. | 24 | n.m. |
| 90 % | 9 | 18,5 | n.m. | 30 | 20 |
| 85 % | 10 | 21 | n.m. | 35 | n.m. |
| 80 % | 11 | 23 | n.m. | 38 | 25 |
| Quantité globale de CFC-113 transformé avant d'atteindre un taux de conversion de 80 % (g/cm3 catalyseur) | 42 | 88 | 140 | 144 | 90 |
| n.m. = non mesuré | | | | | |

## Revendications

1. Procédé pour la préparation de chlorotrifluoroéthylène et de trifluoroéthylène par réaction en phase gazeuse de 1,1,2-trichloro-1,2,2-trifluoroéthane en présence d'hydrogène et d'une composition catalytique comprenant un support à base de carbone sur lequel sont déposés du cuivre et au moins un métal du groupe VIII du tableau périodique des éléments, caractérisée par une teneur pondérale en cuivre dans la composition catalytique de 12 à 22 %.

2. Procédé selon la revendication 1, dans lequel la teneur pondérale en cuivre est de 13,5 à 20 %.

3. Procédé selon la revendication 2, dans lequel la teneur pondérale en cuivre est de 16 à 19 %.

4. Procédé selon une quelconque des revendications 1 à 3, dans lequel le métal du groupe VIII du tableau périodique des éléments est choisi parmi le ruthénium, le rhodium, l'iridium, le platine, le palladium et leurs mélanges.

5. Procédé selon la revendication 4, dans lequel le métal du groupe VIII du tableau périodique des éléments est choisi parmi le platine, le palladium et leurs mélanges.

6. Procédé selon une quelconque des revendications 1 à 5, dans lequel la composition catalytique comprend de 0,05 à 10 % en poids de métal du groupe VIII du tableau périodique des éléments par rapport au poids total de la composition catalytique.

7. Procédé selon la revendication 6, dans lequel la composition catalytique comprend de 1 à 5 % en poids de métal du groupe VIII du tableau périodique des éléments par rapport au poids total de la composition catalytique.

8. Procédé selon la revendication 1, caractérisé en ce que le rapport volumique entre l'hydrogène et le 1,1,2-trichloro-1,2,2-trifluoroéthane mis en oeuvre est compris entre 1 et 7.

9. Composition catalytique comprenant un support à base de carbone sur lequel sont déposés de 12 à 19 % en poids de cuivre par rapport au poids total de la composition catalytique et au moins un métal du groupe VIII du tableau périodique des éléments.

10. Composition catalytique selon la revendication 9 comprenant un support à base de carbone sur lequel sont déposés de 16 à 19 % en poids de cuivre par rapport au poids total de la composition catalytique.

## Patentansprüche

1. Verfahren zur Herstellung von Chlortrifluorethylen und von Trifluorethylen durch Reaktion in der Gasphase von 1,1,2-Trichlor-1,2,2-trifluorethan in Gegenwart von Wasserstoff und einer katalytischen Zusammensetzung, die einen Träger auf Kohlenstoffbasis, auf dem Kupfer und wenigstens ein Metall der Gruppe VIII des Periodensystems der Elemente abgeschieden sind, umfasst, gekennzeichnet durch einen Gewichtsgehalt an Kupfer in der katalytischen Zusammensetzung von 12 bis 22%.

2. Verfahren gemäß Anspruch 1, bei dem der Gewichtsgehalt an Kupfer 13,5 bis 20% beträgt.

3. Verfahren gemäß Anspruch 2, bei dem der Gewichtsgehalt an Kupfer 16 bis 19% beträgt.

4. Verfahren gemäß einem der Ansprüche 1 bis 3, bei dem das Metall der Gruppe VIII des Periodensystems der Elemente unter Ruthenium, Rhodium, Iridium, Platin, Palladium und deren Gemischen ausgewählt ist.

5. Verfahren gemäß Anspruch 4, bei dem das Metall der Gruppe VIII des Periodensystems der Elemente unter Platin, Palladium und deren Gemischen ausgewählt ist.

6. Verfahren gemäß einem der Ansprüche 1 bis 5, bei dem die katalytische Zusammensetzung, bezogen auf das Gesamtgewicht der katalytischen Zusammensetzung, 0,05 bis 10 Gew.-% Metall der Gruppe VIII des Periodensystems der Elemente umfasst.

7. Verfahren gemäß Anspruch 6, bei dem die katalytische Zusammensetzung, bezogen auf das Gesamtgewicht der katalytischen Zusammensetzung, 1 bis 5 Gew.-% Metall der Gruppe VIII des Periodensystems der Elemente umfasst.

8. Verfahren gemäß Anspruch 1, dadurch gekennzeichnet, dass das eingesetzte Volumenverhältnis zwischen Wasserstoff und 1,1,2,-Trichlor-1,2,2-trifluorethan zwischen 1 und 7 liegt.

9. Katalytische Zusammensetzung, die einen Träger auf Kohlenstoffbasis umfasst, auf dem, bezogen auf das Gesamtgewicht der katalytischen Zusammensetzung, 12 bis 19 Gew.-% Kupfer und wenigstens ein Metall der Gruppe VIII des Periodensystems der Elemente abgeschieden sind.

10. Katalytische Zusammensetzung gemäß Anspruch 9, die einen Träger auf Kohlenstoffbasis umfasst, auf dem, bezogen auf das Gesamtgewicht der katalytischen Zusammensetzung, 16 bis 19 Gew.-% Kupfer abgeschieden sind.

## Claims

1. Process for preparing chlorotrifluoroethylene and trifluoroethylene by reaction in the gaseous phase of 1,1,2-trichloro-1,2,2-trifluoroethane in the presence of hydrogen and of a catalytic composition comprising a carbon-based support onto which is deposited copper, and at least one Group VIII metal of the Periodic Table of Elements, characterized by a weight content of copper in the catalytic composition of from 12 to 22 %.

2. Process according to Claim 1, wherein the weight content of copper is from 13.5 to 20 %.

3. Process according to Claim 2, wherein the weight content of copper is from 16 to 19 %.

4. Process according to any one of Claims 1 to 3 wherein the Group VIII metal is selected from the group consisting of ruthenium, rhodium, iridium, platinum, and palladium and the mixtures.

5. Process according to Claim 4, wherein the Group VIII metal is selected from the group consisting of platinum, and palladium and their mixtures.

6. Process according to any one of Claims 1 to 5, wherein the catalytic composition comprises from 0.05 to 10 % by weight of Group VIII metal relative to the total weight of the catalytic composition.

7. Process according to Claim 6, wherein the catalytic composition comprises from 1 to 5 % by weight of Group VIII metal relative to the total weight of the catalytic composition.

8. Process according to Claim 1, characterized in that the volume ratio between the hydrogen and the 1,1,2-trichloro-1,2,2-trifluoroethane is comprised between 1 and 7.

9. Catalytic composition comprising a carbon-based support onto which is deposited from 12 to 19 % by weight of copper, relative to the total weight of the catalytic composition, and at least one Group VIII metal of the Periodic Table of Elements.

10. Catalytic composition according to claim 9, comprising a carbon-based support onto which is deposited from 16 to 19 % by weight of copper relative to the total weight of the catalytic composition.
